# EUROPEAN PATENT SPECIFICATION

(11) **EP 1 543 849 B1**
(45) Date of publication and mention of the grant of the patent: **20.03.2013**
(21) Application number: 04257829.4
(22) Date of filing: 16.12.2004
(51) Int. Cl.: A61L 31/10, A61L 31/16, A61B 17/12

(54) **Activatable bioactive embolic coil**
Aktivierbare bioaktive Emboliespirale
Spirale embolique bioactive pouvant être activée

(30) Priority: 17.12.2003 US 738477
(43) Date of publication of application: 22.06.2005
(73) Proprietor: Codman & Shurtleff, Inc., Raynham, MA 02767 (US)
(72) Inventor: Jones, Donald K., Lauderhill, Florida 33351 (US); Lorenzo, Juan A., Davie, Florida 33328 (US); Pomeranz, Mark L., Davie, Florida 33325 (US); Sherman, Darren, Ft. Lauderdale, Florida 33301 (US)
(74) Representative: Tunstall, Christopher Stephen

(56) References cited:
- WO-A-00/40278
- WO-A-03/064383
- WO-A-03/092791
- US-A1- 2002 082 679
- US-A1- 2002 123 801
- US-A1- 2003 109 824

## Description

### BACKGROUND OF INVENTION

### Field of the Invention

The present invention relates to embolic coils, and more particularly to a emboli coil which includes a bioactive coating placed on the occlusive device for reacting with bodily tissue in order to promote a desired result, such as for example promoting an increase of tissue growth into the occlusive device.

### Description of the Prior Art

For many years medical devices, such as vasculature occlusive devices, have been placed within the vasculature of the human body to occlude, or partially occlude, blood flow through the vasculature. Additionally, such devices have been introduced into aneurysms in order to fill, or partially fill, the aneurysm so as to reduce the pressure which is applied to the interior of the aneurysm in order to prevent further growth or expansion of the aneurysm. These devices may take the form of a coil, such as a helical coil, and are typically placed within the vessel or aneurysm by use of a delivery catheter which is inserted into the vessel and positioned such that the distal end of the delivery catheter is adjacent to a selected site for placement. Once the occlusive device is placed within a blood vessel or aneurysm, surrounding tissue reacts with the "foreign" object and begins to grow into and around the device to provide more complete occlusion of the vessel.

Examples of such delivery catheters are disclosed in U.S. Patent No. 5,108,407, entitled "Method And Apparatus For Placement Of An Embolic Coil" and U.S. Patent No. 5,122,136, entitled "Endovascular Electrolytically Detachable Guidewire Tip For The Electroformation Of Thrombus In Arteries, Veins, Aneurysms, Vascular Malformations And Arteriovenous Fistulas." These patents disclose catheter systems for delivering embolic coils to preselected positions within vessels of the human body in order to treat aneurysms, or alternatively, to occlude a blood vessel at a preselected location.

Occlusive devices which take the form of coils may be helically wound coils, random wound coils, coils wound within coils or other such coil configurations. Examples of various coil configurations are disclosed in U.S. Patent No. 5,334,210, entitled, "Vascular Occlusion Assembly" and U.S. Patent No. 5,382,259, entitled, "Vasoocclusion Coil With Attached Tubular Woven Or Braided Fibrous Covering." Such coils are generally formed from radiopaque metallic materials, such as platinum, gold, tungsten or alloys of these metals. Oftentimes several coils are placed at a given location within a vessel, or within an aneurysm, to more completely occlude, or partially occlude, the flow of blood through the vessel or aneurysm. Thrombus growth onto the coils further enhances the occlusive effect of the coils.

In the past, embolic coils have been placed within the distal end of a delivery catheter and when the distal end of the catheter is properly positioned, the coil may then be pushed out of the end of the catheter with, for example a guidewire, to release the coil at the desired location. This procedure of placement of the embolic coil is conducted under fluoroscopic visualization such that the movement of the coil may be monitored and the coil may be placed at a desired location.

In addition, such coils have been specifically designed to be stretch resistant, such as the vasculature occlusive coil disclosed in U.S. Patent No. 5,853,418, entitled, "Stretch Resistant Vaso-Occlusive Coils (II)" which discloses a helically wound coil having a polymeric stretch resistant member extending through the lumen of the coil and fixedly attached to both ends of the coil to prevent the coil from stretching.

In order to increase the thrombogenicity of an embolic coil, such coils have included a coating, such as collagen, which is applied to the surface of the coil. This concept is disclosed in U.S. Patent No. 5,690,671, entitled, "Embolic Elements And Methods And Apparatus For Their Delivery," which discloses such a collagen coated embolic coil.

WO 00/40278 describes stent grafts coated with an inactive form of a biologically active cooling, which is then activated once the stent graft in deployed. The activation is achieved by injection of a material, like an esterase, into the aneurysm sac.

In addition, U.S. Patent No. 5,980,550, entitled, "Water-Soluble Coating For Bioactive Vasoocclusive Devices," discloses an embolic coil having an inner coating which serves as a thrombogenic agent and an outer coating of a water soluble agent which dissolves after placement of the coil in order expose the thrombogenic inner coating to enhance the growth of thrombus into an around the coil.

The water soluble coating prevents the thrombogenic inner coating from coming into contact with the surrounding blood until the water soluble coating is dissolved by contact with blood which is comprised largely of water.

While the vasculature occlusive device disclosed in this patent includes an agent for enhancing thromboginicity of the device and also includes an outer coating to prevent such activity until the outer coating is dissolved by blood flow, there is no control over when the dissolving process begins and therefore no control over the time in which the thrombogenic agent becomes activated. Without such control, it is possible that thrombus can begin forming on the coil prior to the time the coil is properly placed within a vessel, or aneurysm, therefore making it very difficult if not impossible to reposition, or remove, the improperly placed coil. Alternatively, with water soluble outer protective coating the passive process of removing the outer coating may be so slow that the reaction may not occur in a timely manner.

### SUMMARY OF THE INVENTION

In accordance with the present invention, there is provided an embolic coil as defined in claim 1.

### BRIEF DESCRIPTION OF THE DRAWINGS

Figure 1 is an elevational view of an embolic coil illustrating a medical device in the form of a vascular occlusive coil in accordance with one embodiment of the present invention;
Figure 2 is an elevational view, partly in cross-section of the vascular occlusive coil as shown in Figure 1 illustrating a bioactive coating and an outer barrier coating in accordance with the embodiment of the present invention;
Figures 3A through 3C illustrate the method steps of applying multiple vascular occlusive coils as shown in Figure 1 into an aneurysm and thereafter applying an external agent to thereby activate the embolic coils.

### DESCRIPTION OF THE PREFERRED EMBODIMENT

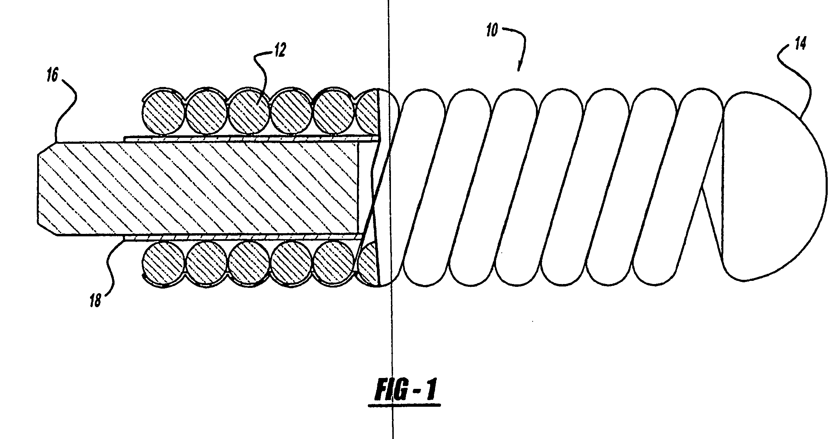

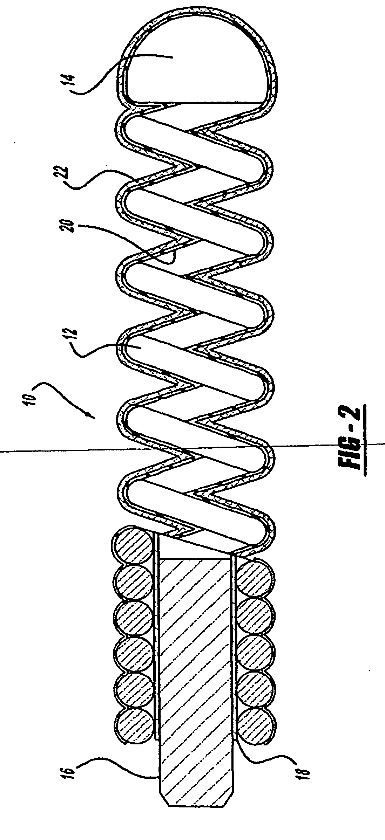

Figures 1 and 2 illustrate a preferred embodiment of a medical device which takes the form of a medical device having an embolic coil 10 which may be placed along with other similar coils into a blood vessel or into an aneurysm in order to partially fill the aneurysm. More particularly, the embolic coil 10 is a typical embolic coil which comprises a helically wound coil 12 formed from a platinum alloy wire wound into a helical configuration. The diameter of the wire is generally in the range of 0.00178 cm (0.0007 inches) to 0,0203 cm (0.008 inches) and the outer diameter of the coil 12 is preferably in a range of 0,00762 cm (0.003 inches) to 0,14 cm (0.055 inches). While the particular embolic coil 12 illustrated in Figures 1 and 2 is shown as being a straight, helically wound coil, it should be appreciated that embolic coils are formed in various configurations and may take the form of a helix, a random shaped configuration or even a coil within a coil.

Preferably the embolic coil 10 includes a weld bead 14 which is attached to the distal end of the coil for providing a less traumatic distal end for the embolic coil 10. In addition, the embolic coil 10 includes a cylindrical headpiece 16 which is placed into the lumen of the helically wound coil 12 at the proximal end of the coil and is held in place by an adhesive material 18 interposed between the cylindrical headpiece 16 and the helical coil 12. The construction of the embolic coil 10 and an associated hydraulic deployment system for placing the embolic coil within an aneurysm is disclosed in more detail in U.S. Patent Application Serial No. 10/102,154, entitled, "Small Diameter Embolic Coil Hydraulic Deployment System," filed March 19, 2002, assigned to the same assignee of the present invention.

Figure 2 illustrates in more detail a bioactive agent 20, and an outer barrier 22 which is disposed upon the bioactive agent 20 for preventing the activation of the bioactive agent until such time as an election is made to activate the bioactive agent. More particularly, the bioactive agent 20, which may take the form of a thrombogenic agent, i.e. an agent which serves to increase or promote the growth and adhesion of thrombus onto the surface of the embolic coil 10, is coated onto the outer surface of the coil 12. While the bioactive agent may take the form of a thrombogenic agent, it should be understood that the bioactive agent may take any form which would induce a desired reaction bodily tissue. For example, the bioactive agent may serve to cause blood to clot onto the surface of the embolic coil 10, it may serve to enhance the adhesion of thrombus onto the surface of the embolic coil, or it may serve to cause adjacent embolic coils to become bonded to each other through adhesion by components of blood. It should be appreciated that there are many other reactions which might exist between the bioactive agent and bodily tissue which would be desirable.

The outer barrier 22 takes the form of a coating which is disposed upon the bioactive agent 20 and serves to insulate the bioactive agent from adjacent bodily fluid until such time as a decision is made by a physician to activate the outer barrier 22. The outer barrier 22 takes the form of a material which is inert to bodily fluid, but which dissolves and exposes the bioactive agent 20 when the outer barrier 22 is subjected to a liquid external agent.

In a preferred embodiment, the bioactive agent 20 is comprised of polyglycolic acid, the outer barrier 22 is comprised of ethylene vinyl alcohol and the external agent for dissolving the outer barrier 22 is dimethyl sulfoxide (DMSO) which serves to dissolve the outer barrier 22 to thereby expose the bioactive agent 20.

It should be appreciated that there are numerous materials which would serve as a bioactive agent, an outer barrier and an external agent, some of which are indicated hereinafter. It is important, however, that the external agent be inert to bodily fluids, such as being non-water soluble, such as blood, in order to prevent the outer barrier 22 from dissolving and exposing the bioactive agent 20 until such time as an election is made by a physician to activate the outer barrier 22.

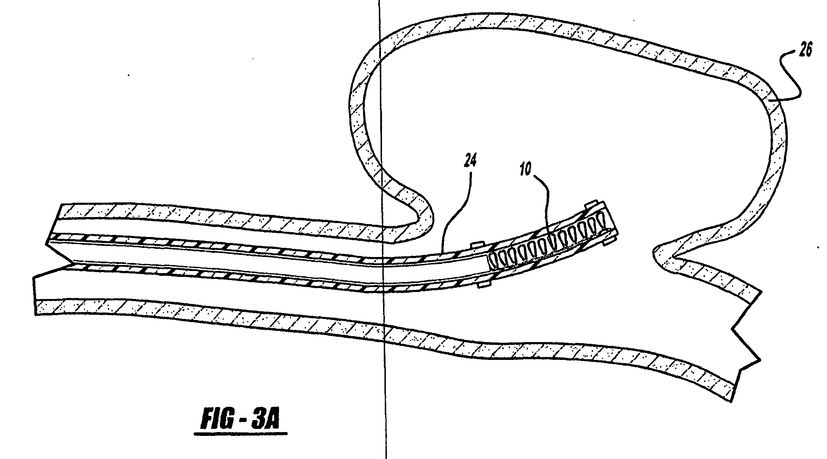

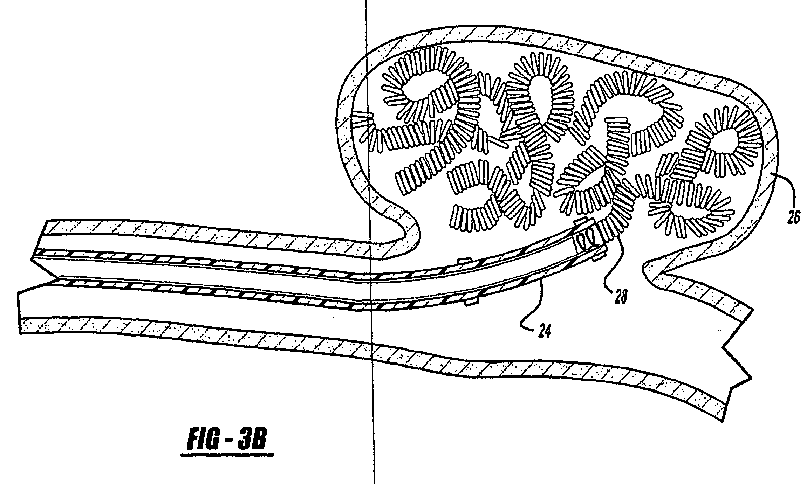

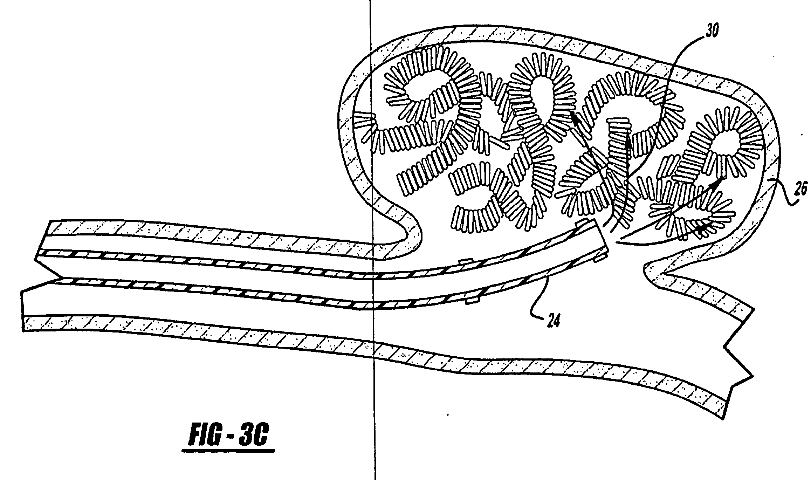

Figures 3A through 3C generally illustrate a method of utilizing the present invention. More particularly, Figure 3A illustrates a delivery catheter 24 having an embolic coil 10 placed in the distal end of the catheter for delivery into an aneurysm 26. Figure 3B illustrates the delivery catheter 24 being used to position multiple vascular occlusive coils including a final embolic coil 28 into the aneurysm 26. Figure 3C illustrates the application of an external agent 30, which may take the form of a solvent for dissolving the outer barrier 22 to thereby activate the outer barrier 22 to expose at least a portion of the bioactive agent 20.

It may be desirable to place all of the vascular occlusive coils into the aneurysm 26 prior to applying the external agent 30, however, another approach is that of placing a single coil into the aneurysm and thereafter activating that single coil, placing a second coil into the aneurysm and thereafter activating the second coil and so forth until all of the coils have been properly placed into the aneurysm. As may be appreciated, the advantage of the subject invention over prior devices is that the physician may determine at what point in time during the process of "filling" an aneurysm the physician elects to activate a coil as opposed to having no control over the time in which the coils become activated.

The bioactive agent may take the form of any material or surface which when placed into the body causes or inhibits a reaction with a bodily substance. For example, the bioactive agent may be a thrombus inducing material, or surface when placed within a blood vessel induces the growth of thrombus onto the surface of the bioactive agent or where the bioactive agent is a thrombolitic agent to inhibit the growth of tissue. The bioactive agent could take the form of a material which causes blood to clot onto the surface of the material, a material which produces an immune response, a material which releases a human growth factor, a material which promotes endothelization, etcetera. Another example of a bioactive agent is a pharmacologic agent which is inactive until the barrier is dissolved a by liquid external source to expose the pharmacological agent to the body. A preferred bioactive agent to be used with a vascular occlusive coil is polyglycolic acid which promotes the growth of tissue.

The outer barrier may take the form of a coating applied to the bioactive agent. A preferred outer barrier to be used with a vascular occlusive coil is a coating of ethylene vinyl alcohol which serves to encase the bioactive agent until a solvent is applied to the coating to thereby dissolve the barrier in order to expose the bioactive agent to the body.

The external agent is a liquid medium which when applied to the outer barrier causes the outer barrier to become ineffective in preventing a reaction between the bioactive agent and bodily tissue. The external agent may take the form of a solvent for dissolving the outer barrier in order to expose the bioactive agent. The external agent may for example be a liquid material for dissolving the outer barrier in order to expose the bioactive agent to bodily tissue. A preferred external agent for an embolic coil is dimethyl sulfoxide which serves as a solvent to dissolve an outer barrier coating comprising ethylene vinyl alcohol so as to permit the bioactive agent to come into contact with bodily tissue. By "bodily tissue" is meant any substance within the body and includes blood, tissue, fibrous growth, etcetera.

## Claims

1. An embolic coil comprising:
a support member;
a bioactive agent disposed on said support member; and,
an outer barrier disposed on said bioactive agent to prevent exposure of said bioactive agent to bodily fluid when said medical device is inserted into a blood vessel, said outer barrier exhibiting the characteristic of being inert to bodily fluid, but dissolving when exposed to an external agent, wherein the external agent is a liquid medium.

2. An embolic coil as defined in Claim 1, wherein the bioactive agent takes the form of a coating applied to the support member.

3. An embolic coil as defined in Claim 1, wherein the bioactive agent is integral with the support member.

4. An embolic coil as defined in Claim 1, wherein the outer barrier takes the form of a coating applied to the bioactive agent.

5. An embolic coil as defined in Claim 2, wherein the outer barrier takes the form of a coating applied to the bioactive agent.

6. An embolic coil as defined in Claim 1, wherein said bioactive agent is comprised of polyglycolic acid and said outer barrier is comprised of ethylene vinyl alcohol.

7. An embolic coil as defined in Claim 6, wherein said external agent is comprised of dimethyl sulfoxide.

8. An embolic coil as defined in Claim 1, wherein said bioactive agent takes the form of a thrombus inducing coating.

9. An embolic coil as defined in Claim 2, wherein said bioactive agent takes the form of a thrombus inducing coating.

10. An embolic coil as defined in Claim 1, wherein said bioactive agent takes the form of a coating which induces the clotting of blood.

11. An embolic coil as defined in Claim 2, wherein said bioactive agent takes the form of a coating which induces the clotting of blood.

## Patentansprüche

1. Emboliewendel, umfassend:
ein Trägerelement;
ein bioaktives Mittel, das auf dem Trägerelement angeordnet ist; und
eine äußere Barriere, die auf dem bioaktiven Mittel angeordnet ist, um Exposition des bioaktiven Mittels gegenüber Körperfluid zu verhindern, wenn die medizinische Einheit in ein Blutgefäß eingeführt wird, wobei die äußere Barriere das Merkmal aufweist, dass sie gegenüber Körperfluid inert ist, sich aber auflöst, wenn sie gegenüber einem externen Mittel exponiert wird, wobei das externe Mittel ein flüssiges Medium ist.

2. Emboliewendel gemäß Anspruch 1, wobei das bioaktive Mittel die Form einer auf das Trägerelement aufgebrachten Beschichtung annimmt.

3. Emboliewendel gemäß Anspruch 1, wobei das bioaktive Mittel mit dem Trägerelement integral ist.

4. Emboliewendel gemäß Anspruch 1, wobei die äußere Barriere die Form einer auf das bioaktive Mittel aufgebrachten Beschichtung annimmt.

5. Emboliewendel gemäß Anspruch 2, wobei die äußere Barriere die Form einer auf das bioaktive Mittel aufgebrachten Beschichtung annimmt.

6. Emboliewendel gemäß Anspruch 1, wobei das bioaktive Mittel aus Polyglycolsäure besteht und die äußere Barriere aus Ethylenvinylalkohol besteht.

7. Emboliewendel gemäß Anspruch 6, wobei das externe Mittel aus Dimethylsulfoxid besteht.

8. Emboliewendel gemäß Anspruch 1, wobei das bioaktive Mittel die Form einer Thrombusinduzierenden Beschichtung annimmt.

9. Emboliewendel gemäß Anspruch 2, wobei das bioaktive Mittel die Form einer Thrombusinduzierenden Beschichtung annimmt.

10. Emboliewendel gemäß Anspruch 1, wobei das bioaktive Mittel die Form einer Beschichtung, die Blutgerinnung induziert, annimmt.

11. Emboliewendel gemäß Anspruch 2, wobei das bioaktive Mittel die Form einer Beschichtung, die Blutgerinnung induziert, annimmt.

## Revendications

1. Spirale d'embolisation, comprenant :
un élément formant support ;
un agent bioactif disposé sur ledit élément formant support ; et
une barrière extérieure, disposée sur ledit agent bioactif, pour empêcher une exposition dudit agent bioactif à un fluide corporel quand ledit dispositif médical est inséré dans un vaisseau sanguin, ladite barrière extérieure présentant la caractéristique d'être inerte vis-à-vis du fluide corporel, mais de se dissoudre après exposition à un agent externe, l'agent externe étant un milieu liquide.

2. Spirale d'embolisation telle que définie dans la revendication 1, dans laquelle l'agent bioactif prend la forme d'un revêtement appliqué sur l'élément formant support.

3. Spirale d'embolisation telle que définie dans la revendication 1, dans laquelle l'agent bioactif est solidaire de l'élément formant support.

4. Spirale d'embolisation telle que définie dans la revendication 1, dans laquelle la barrière extérieure prend la forme d'un revêtement appliqué sur l'agent bioactif.

5. Spirale d'embolisation telle que définie dans la revendication 2, dans laquelle la barrière extérieure prend la forme d'un revêtement appliqué sur l'agent bioactif.

6. Spirale d'embolisation telle que définie dans la revendication 1, dans laquelle ledit agent bioactif est constitué de poly(acide glycolique), et ladite barrière est constituée d'éthylène/alcool vinylique.

7. Spirale d'embolisation telle que définie dans la revendication 6, dans laquelle ledit agent externe est constitué de diméthylsulfoxyde.

8. Spirale d'embolisation telle que définie dans la revendication 1, dans laquelle ledit agent bioactif prend la forme d'un revêtement inducteur de thrombus.

9. Spirale d'embolisation telle que définie dans la revendication 2, dans laquelle ledit agent bioactif prend la forme d'un revêtement inducteur de thrombus.

10. Spirale d'embolisation telle que définie dans la revendication 1, dans laquelle ledit agent bioactif prend la forme d'un revêtement qui induit la coagulation du sang.

11. Spirale d'embolisation telle que définie dans la revendication 2, dans laquelle ledit agent bioactif prend la forme d'un revêtement qui induit la coagulation du sang.
